(19) Europäisches Patentamt
European Patent Office
Office européen des brevets

(11) **EP 3 020 696 B1**

(12) **EUROPÄISCHE PATENTSCHRIFT**

(45) Veröffentlichungstag und Bekanntmachung des Hinweises auf die Patenterteilung:
**03.07.2019   Patentblatt 2019/27**

(51) Int Cl.:
***C01B 3/36*** *(2006.01)*        ***C07C 41/09*** *(2006.01)*

(21) Anmeldenummer: **15003032.8**

(22) Anmeldetag: **22.10.2015**

(54) **VERFAHREN ZUR HERSTELLUNG EINES ODER MEHRERER OXYGENATE**

METHOD FOR THE PRODUCTION OF ONE OR MORE REACTION PRODUCTS

PROCEDE DE FABRICATION D'UN OU PLUSIEURS PRODUITS DE REACTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.11.2014   DE 102014016704**

(43) Veröffentlichungstag der Anmeldung:
**18.05.2016   Patentblatt 2016/20**

(73) Patentinhaber: **Linde Aktiengesellschaft
80331 München (DE)**

(72) Erfinder:
• **Fritz, Helmut
81375 München (DE)**

• **Bartesch, Thomas
85625 Glonn (DE)**
• **Peschel, Andreas
82515 Wolfratshausen (DE)**
• **Fendt, Johannes
81377 München (DE)**

(74) Vertreter: **Fischer, Werner
Linde AG
Technology & Innovation
Corporate Intellectual Property
Dr.-Carl-von-Linde-Straße 6-14
82049 Pullach (DE)**

(56) Entgegenhaltungen:
WO-A1-2011/018233        WO-A2-02/26677
DE-A1- 2 362 944        DE-A1-102012 001 811

**Beschreibung**

Die Erfindung betrifft ein Verfahren und eine Anlage zur Herstellung eines oder mehrerer Reaktionsprodukte, insbesondere Dimethylether oder anderer Oxygenate, gemäß den Oberbegriffen der unabhängigen Patentansprüche.

Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und damit herkömmliche Fluorchlorkohlenwasserstoffe ersetzen. Neuerdings wird Dimethylether auch zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nicht oder nur geringfügig modifiziert werden. Dimethylether verbrennt dabei vergleichsweise sauber und ohne Rußbildung.

Dimethylether kann mittels einer zweistufigen Synthese aus Synthesegas über das Intermediat Methanol hergestellt werden, wie beispielsweise in Kapitel 4 des DME Handbook, Japan DME Forum, Tokyo 2007, ISBN 978-4-9903839-0-9, beschrieben. Eine "zweistufige" Synthese zeichnet sich dadurch aus, dass zunächst Methanol aus Synthesegas hergestellt wird, wobei das Methanol von nicht umgesetztem Synthesegas abgetrennt und das Methanol anschließend separat und in einem weiteren Schritt zu Dimethylether und Wasser dehydratisiert wird.

Die vorliegende Erfindung eignet sich für den Einsatz bei der Herstellung von Dimethylether, aber auch anderer Oxygenate, aus Synthesegas. Das Synthesegas bzw. ein entsprechender Synthesegassammelstrom kann im Rahmen der vorliegenden Erfindung beliebigen bekannten aufbauenden Reaktionen, beispielsweise der Fischer-Tropsch-Synthese, unterworfen werden.

Gemäß einer gängigen Definition, die auch hier Anwendung findet, handelt es sich bei Oxygenaten um Verbindungen, die wenigstens eine kovalent an ein Sauerstoffatom gebundene Alkylgruppe aufweisen. Die wenigstens eine Alkylgruppe kann bis zu fünf, bis zu vier oder bis zu drei Kohlenstoffatome aufweisen. Insbesondere weisen die Oxygenate, die im Rahmen der vorliegenden Erfindung von Interesse sind, Alkylgruppen mit einem oder zwei Kohlenstoffatomen auf, insbesondere handelt es sich um Methylgruppen. Insbesondere handelt es sich um einwertige Alkohole und Dialkylether wie Methanol und Dimethylether oder entsprechende Mischungen.

In der Patentliteratur, beispielsweise der DE 236 29 44 A1, wird bereits 1973 die direkte Synthese von Dimethylether aus Synthesegas beschrieben. Hierbei wird eine gemeinsame Reaktionsstufe verwendet, in der aus Wasserstoff, Kohlenmonoxid und Kohlendioxid Methanol und Dimethylether gebildet werden. Die "direkte" Synthese von Dimethylether aus Synthesegas zeichnet sich dadurch aus, dass hierbei typischerweise keine Abtrennung von Methanol und dessen getrennte weitere Umsetzung zu Dimethylether erfolgt. Entsprechende Verfahren sind beispielsweise der Veröffentlichung "Dimethyl Ether (DME) Technology and Markets", PERP07/08-S3 der Nexant Technology, Inc., Dezember 2008, sowie dem erwähnten DME Handbook des Japan DME Forum zu entnehmen. DE 10 2012 001 811 offenbart ebenfalls ein Verfahren zur Herstellung von DME.

Die Stöchiometriezahl (engl. Stoichiometric Number, SN) ist in der Fachwelt zur Charakterisierung von Synthesegas üblich. Sie ist definiert als

$$SN = (xH_2 - xCO_2) / (xCO + xCO_2),$$

wobei x jeweils für den molaren Gehalt der Komponenten Wasserstoff ($H_2$), Kohlenmonoxid (CO) und Kohlendioxid ($CO_2$) steht. Bei entsprechenden zweistufigen Synthesen werden typischerweise Synthesegase mit Stöchiometriezahlen von 2 bis 2,05 als Frischeinsatz (Makeup) für die Methanolsynthese verwendet, bei direkten Synthesen werden dagegen oft deutlich niedrigere Stöchiometriezahlen eingesetzt.

In den klassischen Verfahren zur einstufigen Synthese von Dimethylether werden beispielsweise Stöchiometriezahlen von 1 bis 1,5 gefordert, wobei i.d.R. vor der Durchführung eines entsprechenden Verfahrens Kohlendioxid aus dem Synthesegas abgetrennt werden sollte. Ein durch die Anmelderin entwickeltes Verfahren, bei dem Dimethylether aus methanreichem Erdgas direkt synthetisiert wird, umfasst hingegen die Verwendung eines Synthesegases mit einer Stöchiometriezahl von 2 bis 5. Diese kann beispielsweise durch einen Frischeinsatz (Makeup) an Synthesegas mit einer Stöchiometriezahl von ca. 2,05 und ein Rückführen des jeweils nicht umgesetzten Synthesegases erreicht werden, wodurch sich Wasserstoff im Prozess anreichert und sich damit die Stöchiometriezahl erhöht. Die genannte Stöchiometriezahl von 2 bis 5 ergibt sich hierbei am Eintritt in den verwendeten Synthesereaktor.

Zur Bereitstellung eines Synthesegases mit einer Stöchiometriezahl von 2 bis 2,05 aus methanreichem Erdgas sind unterschiedliche Verfahren bekannt. Beispielsweise schlägt die US 2009/0105356 A1 zur Herstellung eines Synthesegases eine Kombination einer Dampfreformierung und einer autothermen Reformierung in Reihenschaltung vor, wobei ein Teil des Einsatzes direkt der autothermen Reformierung zugeführt und an der Dampfreformierung vorbeigeleitet wird. Beispielsweise aus der US 7,485,767 B2 ist ein Verfahren bekannt, bei dem Erdgas einerseits einer partiellen Oxidation oder einer autothermen Reformierung und andererseits einer Dampfreformierung zugeführt wird. Die partielle Oxidation bzw. die autotherme Reformierung einerseits und die Dampfreformierung andererseits sind also parallel angeordnet. Die jeweils gewonnenen Synthesegase werden zu einem Synthesegassammelstrom vereinigt, der die gewünschte Stöchiometriezahl aufweist.

Die partielle Oxidation bzw. die autotherme Reformierung wird dabei bei einem höheren Druck durchgeführt als die Dampfreformierung, so dass das in der Dampfreformierung erhaltene Synthesegas vor der Vereinigung mit dem in der partiellen Oxidation bzw. der autothermen Reformierung enthaltenen Synthesegas nachverdichtet werden muss. Auch alternative Verfahren sind bekannt und beispielsweise in der WO 1993/015999 A1 und der EP 0 522 744 A2 beschrieben. Prozessrechnungen lassen erwarten, dass insbesondere eine Parallelschaltung von partieller Oxidation oder autothermer Reformierung einerseits und Dampfreformierung andererseits zu besonders hoher Energieeffizienz führen. Die Energieeffizienz wird dabei insbesondere dann gesteigert, wenn die partielle Oxidation bzw. autotherme Reformierung einerseits bei vergleichsweise hohem Druck (beispielsweise 3,5 bis 8 MPa (35 bis 80 bar)) und die Dampfreformierung andererseits bei vergleichsweise niedrigem Druck (beispielsweise ca. 1 bis 3,5 MPa (10 bis 35 bar)) durchgeführt wird.

Bei der direkten Synthese von Dimethylether baut sich, wenn nicht umgesetztes Synthesegas zur Synthese zurückgeführt wird, eine hohe Kohlendioxidkonzentration im Synthesegas auf. Dies kann zu verringerten Reaktionsgeschwindigkeiten und geringerem Umsatz führen. Ferner können bei der direkten Synthese von Dimethylether vergleichsweise große Mengen von Nebenprodukten wie Methan, Ethan und Propan entstehen, die aufwendig abgetrennt werden müssen. Entsprechendes gilt auch für andere aufbauende Reaktionen.

Die vorliegende Erfindung stellt sich die Aufgabe, entsprechende Verfahren insbesondere bezüglich der letztgenannten Aspekte zu verbessern.

Offenbarung der Erfindung

Diese Aufgabe wird durch ein Verfahren zur Herstellung eines oder mehrerer Reaktionsprodukte, insbesondere Dimethylether oder anderer Oxygenate, mit den Merkmalen des Anspruchs 1 gelöst. Bevorzugte Ausgestaltungen sind Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Zu Verfahren und Vorrichtungen zur Herstellung von Synthesegas, insbesondere bezüglich der partiellen Oxidation (engl. Partial Oxidation), der autothermen Reformierung (Autothermal Reforming, ATR) und der Dampfreformierung (Steam Methane Reforming, SMR) sei auf einschlägige Lehrbuchartikel wie beispielsweise den Artikel "Gas Production" in Ullmann's Encyclopedia of Industrial Chemistry, Onlineausgabe 15. Dezember 2006, DOI 10.1002/14356007.a12_169.pub2, oder den Abschnitt 4.2, "Synthesis gas production technology" des DME Handbook verwiesen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise $\pm$ 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Flüssige und gasförmige Ströme können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Ist hier davon die Rede, dass ein Strom "aus Fluid" eines anderen Stroms gebildet wird, sei hierunter sowohl verstanden, dass zur Bildung des weiteren Stroms sämtliches Fluid des Ausgangsstroms verwendet wird, es kann jedoch auch nur ein Teil eines entsprechenden Ausgangsstroms, beispielsweise nach Abtrennung anderer Komponenten, beispielsweise von Kondensaten, verwendet werden.

Ein "Verdichter" ist im hier verwendeten Sprachgebrauch eine Vorrichtung, die zum Verdichten wenigstens eines gasförmigen Stroms von wenigstens einem Eingangsdruck, bei dem dieser dem Verdichter zugeführt wird, auf wenigstens einen Enddruck, bei dem dieser dem Verdichter entnommen wird, eingerichtet ist. Ein Verdichter bildet eine bauliche Einheit, die jedoch mehrere "Verdichterstufen" in Form von Kolben-, Schrauben- und/oder Schaufelrad- bzw. Turbinenanordnungen (also Axial- oder Radialverdichterstufen) aufweisen kann. Insbesondere werden entsprechende Verdichterstufen mittels eines gemeinsamen Antriebs, beispielsweise über eine gemeinsame Welle, angetrieben.

Vorteile der Erfindung

Am Eintritt eines Reaktors zur Umsetzung des Synthesegases, also zur Durchführung einer aufbauenden Reaktion wie zuvor erläutert, erweist sich eine Stöchiometriezahl von 2 oder mehr als 2 als vorteilhaft. Die zuvor erläuterten klassischen Verfahren zur Direktsynthese von Dimethylether arbeiten mit Synthesegasen, in denen das Verhältnis von Wasserstoff zu Kohlenmonoxid 1 beträgt und die typischerweise kein oder kaum Kohlendioxid aufweisen. Dadurch liegt dort ein sehr hoher Umsatz vor, allerdings werden auch vergleichsweise große Mengen an Kohlendioxid gebildet. Durch die Bildung von Kohlendioxid verschlechtern sich die Energieeffizienz und das Emissionsverhalten entsprechender Anlagen.

Wie erfindungsgemäß erkannt wurde, ist ein geringer Anteil von Kohlendioxid vorteilhaft, weil hierdurch beispielsweise ein höherer Umsatz im Reaktor möglich ist. Dies ist zunächst thermodynamisch nicht zu erwarten, da der höchste thermodynamisch mögliche Umsatz mit einem kohlendioxidfreien Gas erfolgt. Durch eine schnellere Reaktionskinetik in Anwesenheit von Kohlendioxid erhöht sich jedoch der Umsatz im Reaktor. Von daher ist es besonders wichtig, den Anteil von Kohlendioxid am Reaktoreintritt genau einzustellen. Hierzu, und um eine gewünschte Stöchiometriezahl von beispielsweise 2 zu erzielen, ist eine Kombination von partieller Oxidation bzw. autothermer Reformierung und SMR besonders effizient.

Zudem wird der Aufbau von inerten Komponenten mit Siedepunkten zwischen Dimethylether (oder einem anderen gebildeten Oxygenat oder Reaktionsprodukt der Synthesegasumsetzung) und Kohlendioxid im Synthesegaskreislauf um einen entsprechenden Reaktor vermieden, da diese Komponenten ebenfalls in die Dampfreformierung zurückgeführt und dort zu Synthesegas umgesetzt werden.

Die vorliegende Erfindung schlägt daher ein Verfahren zur Herstellung eines oder mehrerer Reaktionsprodukte einer aufbauenden Reaktion, beispielsweise von Dimethylether oder anderer Oxygenate, vor, bei dem ein erster methanreicher Einsatzstrom einem partiellen Oxidationsverfahren und/oder einem autothermen Reformierungsverfahren und ein zweiter methanreicher Einsatzstrom einem Dampfreformierungsverfahren unterworfen wird. Insoweit gleicht das erfindungsgemäße Verfahren jenem, wie es zuvor beispielsweise unter Bezugnahme auf die US 7,485,767 B2 beschrieben wurde. Die Dampfreformierung kann insbesondere eine Vorreformierung (engl. Pre-Reforming) umfassen. Ebenso wird, wie insoweit bekannt, mittels des partiellen Oxidationsverfahrens und/oder des autothermen Reformierungsverfahrens ein erster Synthesegas enthaltender Abstrom und mittels des Dampfreformierungsverfahrens ein zweiter Synthesegas enthaltender Abstrom gebildet. Ist hier davon die Rede, dass ein "Synthesegas enthaltender" Abstrom gebildet wird, sei hierunter verstanden, dass ein Abstrom gebildet wird, der Wasserstoff, Kohlendioxid und Kohlenmonoxid in den typischerweise in Synthesegas vorzufindenden Mengenanteilen aufweist. Diese Mengenanteile lassen sich, wie zuvor erläutert, günstigerweise über die Stöchiometriezahl definieren.

Die Erfindung sieht ferner vor, aus Synthesegas des ersten Abstroms und Synthesegas des zweiten Abstroms einen Synthesegassammelstrom zu bilden und Fluid des Synthesegassammelstroms in einem Syntheseeinsatzstrom unter Erhalt eines Kohlendioxid und wenigstens ein Reaktionsprodukt enthaltenden Syntheseabstroms einer aufbauenden Reaktion, beispielsweise der Direktsynthese von Dimethylether, zu unterwerfen. Die in dem erhaltenen Syntheseabstrom enthaltenen Verbindungen werden teilweise in der aufbauenden Reaktion, beispielsweise dem Verfahren zur Direktsynthese von Dimethylether, gebildet, teilweise handelt es sich um nicht umgesetzte Verbindungen des Syntheseeinsatzstroms.

Wie zuvor erläutert, ist eine Direktsynthese von Dimethylether eine Synthese, bei der kein Methanol separat gewonnen und weiter zu Dimethylether umgesetzt wird, es werden vielmehr direkt in einem einzigen Reaktionsschritt Dimethylether und gegebenenfalls Methanol gebildet. Ein "Reaktionsschritt" ist dabei nicht als molekularer Reaktionsschritt, sondern als technischer Reaktionsschritt zu verstehen. Dieser technische Reaktionsschritt zeichnet sich dadurch aus, dass er ohne Zwischenproduktabtrennung in einem oder mehreren Reaktoren durchgeführt wird, kann aber mehrere molekulare Reaktionsschritte umfassen. Der Syntheseabstrom enthält bei der Direktsynthese von Dimethylether neben den erwähnten Komponenten Kohlendioxid und Dimethylether auch Methanol, nicht umgesetztes Synthesegas sowie Nebenprodukte der Dimethylethersynthese, wie sie zuvor erläutert wurde, insbesondere Kohlenwasserstoffe und Inertgase wie Methan, Ethan und Stickstoff. Entsprechende Verbindungen können auch in anderen aufbauenden Reaktionen, wie eingangs erläutert, erhalten werden. Eine "aufbauende Reaktion" ist eine Reaktion, bei der aus den niedermolekularen Verbindungen des Synthesegases, insbesondere Kohlenmonoxid und Wasserstoff, zumindest teilweise höhermolekulare Verbindungen wie Oxygenate oder Kohlenwasserstoffe gebildet werden.

Wie zuvor erläutert, ist es in bekannten Verfahren, die die erläuterten Schritte umfassen, von Nachteil, dass sich bei einem Recycle des nicht umgesetzten Synthesegases aus dem Syntheseabstrom Kohlendioxid in einem entsprechenden Verfahren anreichern und damit die Dimethylethersynthese oder andere aufbauende Reaktionen hemmen kann. Die Erfindung sieht daher vor, aus Fluid des Syntheseabstroms zumindest einen kohlendioxidreichen ersten Recyclestrom zu bilden und Fluid des ersten Recyclestroms dem Dampfreformierungsverfahren zu unterwerfen. Durch die Entnahme des kohlendioxidreichen ersten Recyclestroms wird der Kohlendioxidgehalt des verbleibenden, nicht umgesetzten Synthesegases in dem Syntheseabstrom verringert. Die Rückführung des kohlendioxidreichen ersten Recyclestroms in die Dampfreformierung ist besonders günstig, weil die Abtrennung des kohlendioxidreichen ersten Recyclestroms bei etwas höherem Druck als die Dampfreformierung erfolgen kann. Das Kohlendioxid kann daher ohne gesonderte Verdichtung der Dampfreformierung zugeführt und dort effizient zu Kohlenmonoxid umgesetzt werden. Insgesamt wird dadurch ohne zusätzlichen Verdichter die Kohlenstoffeffizienz gesteigert und der Umsatz in der aufbauenden Reaktion erhöht.

Das verbleibende Synthesegas, das ebenfalls aus dem Syntheseabstrom abgetrennt wird bzw. nach der Abtrennung weiterer Komponenten, insbesondere von Reaktionsprodukten der aufbauenden Reaktion wie Dimethylether, erhalten wird, weist aufgrund der erfindungsgemäß vorgeschlagenen Maßnahmen einen vergleichsweise geringen Kohlendioxidgehalt auf, so dass sich der aufbauenden Reaktion, in die dieses zurückgeführt wird, insbesondere ein wasserstoffreiches und vergleichsweise kohlendioxidarmes Synthesegas zuführen lässt. Dieses wird aus dem Synthesegas des Synthesegassammelstroms und eines entsprechenden Recyclestroms gebildet.

Erfindungsgemäß ist also vorteilhafterweise vorgesehen, aus Fluid des Syntheseabstroms einen Synthesegas enthaltenden und an Kohlendioxid abgereicherten zweiten Recyclestrom zu bilden, wobei Fluid des zweiten Recyclestroms dem Syntheseeinsatzstrom zugespeist wird. Wie bereits erläutert, lassen sich durch die Bildung des kohlendioxidreichen ersten Recyclestroms die Kohlendioxidgehalte in einem entsprechenden zweiten Recyclestrom verringern.

Besondere Vorteile ergeben sich, wenn das Synthesegas, das als sogenannter Makeupstrom aus dem Synthesegas des ersten Abstroms und des zweiten Abstroms gebildet wird, eine Stöchiometriezahl von 1,5 bis 2 aufweist. Ein "Makeupstrom" ist dabei der Synthesegassammelstrom, aus dem zusammen mit dem zweiten Recyclestrom der Syntheseeinsatzstrom gebildet wird. Hierdurch kann erreicht werden, dass der Syntheseeinsatzstrom eine Stöchiometriezahl von 2 bis 3 aufweist.

Die vorliegende Erfindung lässt sich also gemäß der erläuterten Ausführungsform dadurch charakterisieren, dass eine Parallelschaltung von partieller Oxidation bzw. autothermer Reformierung und Dampfreformierung vorgenommen wird, wobei ein aus einer der aufbauenden Reaktion, beispielsweise der Dimethylethersynthese, nachgeschalteten Trennsequenz entnommener kohlendioxidreicher Strom als ("erster") Recyclestrom in die Dampfreformierung zurückgeführt wird. Die genannten Verfahren können aufgrund der vorliegenden Erfindung ein Synthesegas mit einer Stöchiometriezahl von unter 2 liefern, wohingegen in herkömmlichen Verfahren typischerweise frisch eingesetztes Synthesegas durch eine Stöchiometriezahl von 2 bis 2,05 gekennzeichnet ist.

In entsprechenden Verfahren wird vorteilhafterweise aus dem Fluid des Syntheseabstroms, wie bei aufbauenden Reaktionen grundsätzlich bekannt, ein an wenigstens einem Reaktionsprodukt der aufbauenden Reaktion, beispielsweise Dimethylether, angereicherter Produktstrom gebildet und dem Verfahren entzogen. Die vorliegende Erfindung ermöglicht insbesondere die Synthese von Dimethylether in gegenüber dem Stand der Technik wirtschaftlicherer Weise, da durch die Verringerung des Kohlendioxidgehalts bzw. die Einstellung einer geeigneten Stöchiometriezahl des Syntheseeinsatzstroms günstige Reaktionsbedingungen erhalten werden.

Ein weiterer besonders vorteilhafter Aspekt der vorliegenden Erfindung ist, dass durch die Entfernung von Kohlendioxid aus einem direkt in die aufbauende Reaktion, beispielsweise in die Dimethylethersynthese, zurückgeführten Recyclestrom das Kohlenmonoxid/Kohlendioxid-Verhältnis erhöht und der Reaktionsumsatz gesteigert wird. Hierdurch sinken die Volumina, die in entsprechenden Recycleströmen zu bewältigen sind, und ein Reaktor zur Durchführung der aufbauenden Reaktion und ein anschließender Trennteil können kleiner ausgeführt werden. Die Erfindung ermöglicht gleichzeitig die Entfernung von inerten Komponenten wie Methan und Ethan aus dem Prozess, die in der Dampfreformierung in geeigneter Weise behandelt werden können.

Der erste Recyclestrom wird vorteilhafterweise mit einem Gehalt von wenigstens 60, 80 oder 90 Molprozent Kohlendioxid gebildet und weist im Übrigen typischerweise Komponenten mit einem Siedepunkt unterhalb wenigstens eines in der aufbauenden Reaktion gebildeten Reaktionsprodukts, insbesondere unterhalb von Dimethylether, auf, beispielsweise Ethan. Das abgetrennte Ethan kann mit besonderem Vorteil in der Dampfreformierung genutzt werden und ist in dem ersten Recyclestrom enthalten. Ein besonderer Vorteil der vorliegenden Erfindung ist dabei, dass Kohlendioxid und Komponenten wie Ethan aus dem Syntheseabstrom in bereits vorhandenen Trenneinrichtungen ohne zusätzliches Equipment zur Druckwechselabsorption oder Membranverfahren entfernt werden können.

Die Abtrennung von Reaktionsprodukten der aufbauenden Reaktion, beispielsweise von Dimethylether, aus dem Syntheseabstrom erfolgt typischerweise mittels Absorption oder Kondensation und Destillation. Im ersten Fall (Absorption) werden physikalische Waschmittel wie Methanol verwendet, um das oder die Reaktionsprodukte, beispielsweise Dimethylether, vom Rest des Syntheseabstroms abzutrennen. Dabei löst sich auch eine gewisse Menge an Kohlendioxid im Waschmittel und muss nach der Regenerierung des Waschmittels wieder von dem oder den Reaktionsprodukten abgetrennt werden. Im zweiten Fall (Kondensation und Destillation) wird der Syntheseabstrom auf eine Temperatur abgekühlt, bei der das oder die Reaktionsprodukte, beispielsweise Dimethylether, und schwerere Komponenten auskondensieren. Dabei kondensiert auch ein Teil des Kohlendioxids. Das auf diese Weise erhaltene Kondensat wird destillativ aufgetrennt und es entsteht der kohlendioxidreiche erste Recyclestrom ohne zusätzliche Trenneinheit. Dieser enthält in beiden Fällen auch andere leichtere Verbindungen. Beide Verfahren können auch kombiniert werden. Sie haben gemeinsam, das zur Aufreinigung des oder der Reaktionsprodukte, beispielsweise Dimethylether, eine Kolonne, die Kohlendioxid von dem oder den Reaktionsprodukten abtrennt, benötigt wird. Diese kann vorteilhaft zwischen 1 und 3 MPa (10 und 30 bar) betrieben werden, so dass, wie erläutert, entsprechende Komponenten ohne zusätzliche Verdichtung in die Dampfreformierung geführt werden können.

Komponenten wie Ethan werden somit aus dem Synthesegasloop um den Reaktor zur Durchführung der aufbauenden Reaktion abgetrennt und in der Dampfreformierung zu Synthesegas umgewandelt. Herkömmlicherweise verhalten sich diese Komponenten im Synthesegasloop um den Reaktor inert und verringern so den Partialdruck der Reaktanden bzw. Edukte. Dadurch wird der Umsatz reduziert und die Volumenströme werden in dem gesamten Synthesegasloop um den Reaktor zusätzlich erhöht, was sich nachteilig auswirkt.

Mit besonderem Vorteil wird der erste Recyclestrom bei einem Druck von ca. 1 bis 3 MPa (10 bis 30 bar) bereitgestellt, sodass dieser, wie auch nachfolgend erläutert, einen geeigneten Druck aufweist, um ohne zusätzliche Verdichtung direkt in das Dampfreformierungsverfahren rezykliert werden zu können.

Vorteilhafterweise weist der zweite Recyclestrom überwiegend Wasserstoff, Kohlendioxid und Kohlenmonoxid auf, wobei der Gehalt an Kohlendioxid bei 1 bis 15 Molprozent liegt, also deutlich geringer sein kann, als jener herkömmlicher Recycleströme, die in bekannten Verfahren zur Synthese von Dimethylether in die Synthesestufe zurückgeführt werden.

Gemäß einer besonders bevorzugten Ausgestaltung des erfindungsgemäßen Verfahrens wird der erste Abstrom auf einem ersten Druckniveau und der zweite Abstrom auf einem zweiten Druckniveau, das geringer als das erste Druckniveau ist, bereitgestellt, wobei vor dem Bilden des Synthesegassammelstroms das Synthesegas des zweiten Abstroms auf das erste Druckniveau verdichtet wird. Besonders vorteilhaft sind in diesem Zusammenhang die zuvor erläuterten Druckniveaus, wobei das erste Druckniveau insbesondere bei 3 bis 10 MPa (30 bis 100 bar), vorteilhafterweise bei 3,5 bis 8 MPa (35 bis 80 bar), und das zweite Druckniveau insbesondere bei 0,5 bis 4 MPa (5 bis 40 bar), vorteilhafterweise bei 1 bis 3,5 MPa (10 bis 35 bar), liegt. Die genannten Druckniveaus ermöglichen besonders vorteilhafte Reaktionsbedingungen, gleichzeitig erlaubt ein Betrieb des Dampfreformierungsverfahrens bei dem zweiten Druckniveau eine einfache Rückführung des ersten Recyclestroms ohne zusätzliche Apparate.

Vorteilhafterweise werden im Rahmen des erfindungsgemäßen Verfahrens der erste Einsatzstrom und der zweite Einsatzstrom aus Fluid eines gemeinsamen Ausgangsstroms gebildet, beispielsweise aus einem Erdgasstrom, der auf dem ersten und dem zweiten Druckniveau bereitgestellt und zuvor oder anschließend erwärmt und entschwefelt wird. Die gemeinsame Behandlung des ersten und des zweiten Einsatzstroms durch Erwärmen und Entschwefeln ermöglicht ein besonders effizientes Verfahren. Vorteilhafterweise wird dabei zum Erwärmen Abwärme des zweiten Abstroms, d.h. des Abstroms der Dampfreformierung, verwendet.

Die Erfindung wird nachfolgend unter Bezugnahme auf die beigefügte Figur näher erläutert, welche eine bevorzugte Ausführungsform der Erfindung zeigt.

Kurze Beschreibung der Zeichnungen

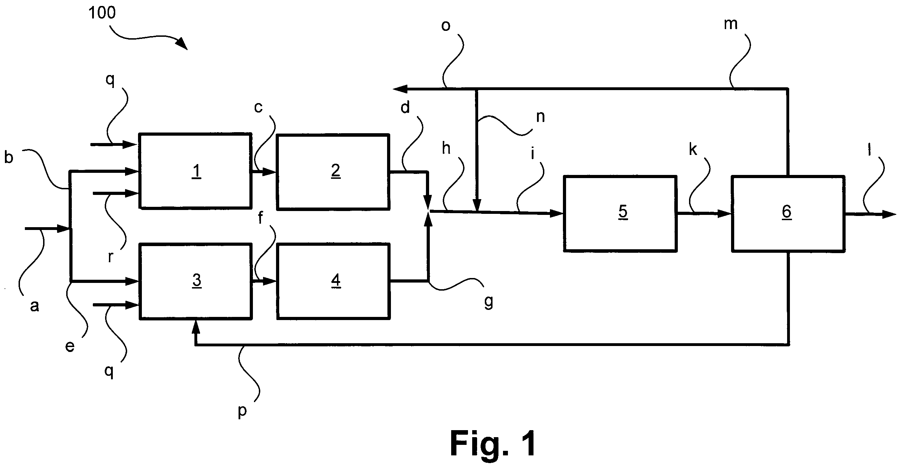

Figur 1 veranschaulicht eine Anlage zur Durchführung der Erfindung in Form eines schematischen Ablaufplans. Ausführliche Beschreibung der Zeichnungen

In Figur 1 ist eine Anlage zur Durchführung des erfindungsgemäßen Verfahrens in Form eines schematischen Ablaufplans dargestellt und insgesamt mit 100 bezeichnet. Die Figur 1 veranschaulicht gleichzeitig Schritte eines entsprechenden Verfahrens, so dass, wenn von einer "partiellen Oxidation" bzw. "autothermen Reformierung", einer "Dampfreformierung", einer "Synthesegasaufbereitung" usw. oder von einem "Verfahren zur Synthese von Dimethylether" die Rede ist, entsprechende Anlagenkomponenten, beispielsweise Reaktoren, aber auch entsprechende Verfahrensschritte umfasst sein können. Die Dampfreformierung bzw. ein oder mehrere entsprechender Reaktoren können insbesondere eine Vorreformierung (engl. Pre-Reforming) bzw. entsprechende Reaktoreinheiten umfassen.

Der Anlage 100 wird ein Ausgangsstrom a, beispielsweise erwärmtes und/oder entschwefeltes Erdgas, zugeführt. Fluid des Ausgangsstroms a kann beispielsweise auf einem ersten und einem zweiten Druckniveau, bei dem dieses einer partiellen Oxidation bzw. einer autothermen Reformierung 1 einerseits und einer Dampfreformierung 3 andererseits (siehe unten) zugeführt wird, bereitgestellt werden, wobei vor oder nach der Bereitstellung auf entsprechenden Druckniveaus eine gemeinsame oder eine getrennte Erwärmung und Entschwefelung erfolgt. Die Erwärmung kann beispielsweise mittels Abwärme der Dampfreformierung 3 bzw. eines entsprechend erhaltenen Stroms erfolgen.

Aus Fluid des Ausgangsstroms a wird ein erster Einsatzstrom b auf einem Druckniveau von beispielsweise 30 bis 100 bar ("erstes" Druckniveau) der partiellen Oxidation oder autothermen Reformierung 1 zugeführt. Der partiellen Oxidation bzw. autothermen Reformierung 1 werden ferner ein Dampfstrom q und ein Sauerstoffstrom oder sauerstoffreicher Strom r zugeführt. Mittels der partiellen Oxidation bzw. autothermen Reformierung 1 wird ein Abstrom c ("erster" Abstrom) erhalten und einer Synthesegasaufbereitung 2 zugeführt. Die Synthesegasaufbereitung 2 kann beispielsweise einen Scrubber umfassen. Stromab der Synthesegasaufbereitung 2 liegt ein erster Synthesegasstrom d auf dem zuvor erläuterten ersten Druckniveau vor.

Ein zweiter aus dem Fluid des Ausgangsstroms a gebildeter Einsatzstrom e wird der Dampfreformierung 3 zugeführt, der ferner ein Dampfstrom q und ein unten erläuterter Recyclestrom ("erster" Recyclestrom) p zugeführt wird. Durch die Dampfreformierung 3 wird ein Abstrom f ("zweiter" Abstrom) erhalten und ebenfalls einer Synthesegasaufbereitung, hier mit 4 bezeichnet, zugeführt. Stromab der Synthesegasaufbereitung 4 liegt ein Synthesegasstrom g ("zweiter" Synthesegasstrom) vor.

Der zweite Synthesegasstrom g weist typischerweise einen geringeren Druck als der erste Synthesegasstrom d auf, weil die Dampfreformierung 3, wie zuvor erläutert, typischerweise auf einem geringerem Druckniveau (dem "zweiten" Druckniveau) betrieben wird. Der zweite Synthesegasstrom g wird daher verdichtet (nicht gezeigt). Die Synthesegasströme d und g, bzw. auch nur gewisse Anteile entsprechender Synthesegasströme d und g, werden anschließend zu einem Synthesegassammelstrom h vereinigt. Zu dem Synthesegassammelstrom h wird ferner ein Recyclestrom ("zweiter" Recyclestrom) n, der unten erläutert wird, zugespeist, wodurch ein Syntheseeinsatzstrom i gebildet wird.

Der Syntheseeinsatzstrom i wird einer aufbauenden Reaktion 5, beispielsweise zur Direktsynthese von Dimethylether, zugeführt, mittels derer ein Syntheseabstrom k erhalten wird, der, wie erwähnt, neben dem oder den Zielprodukten, beispielsweise Dimethylether, nicht umgesetztes Synthesegas sowie weitere Nebenprodukte wie beispielsweise Methanol, Stickstoff, Methan und Ethan enthält. Der Syntheseabstrom k bzw. Fluid eines entsprechenden Syntheseabstroms k wird einer Trenneinheit 6 zugeführt, in der unter anderem ein an einem oder mehreren Reaktionsprodukten der aufbauenden Reaktion 5, beispielsweise Dimethylether, reicher Strom l als Produktstrom erhalten und aus einem entsprechenden Verfahren ausgeführt wird.

In der Trenneinheit 6 werden ferner der erwähnte erste Recyclestrom p und ein weiterer Strom m gebildet. Der erste Recyclestrom p ist, wie erwähnt, reich an Kohlendioxid, und weist insbesondere 60 bis 100 Molprozent Kohlendioxid auf. In den ersten Recyclestrom p gehen auch Inertgase mit einem Dampfdruck unterhalb des wenigstens einen Reaktionsprodukts, beispielsweise Dimethylether, über, beispielsweise Ethan. Dieser erste Recyclestrom p wird in die Dampfreformierung 3 rezykliert. Er weist einen Druck von typischerweise circa 10 bis 30 bar auf, sodass er ohne weitere Verdichtung in die Dampfreformierung 3 zurückgeführt werden kann. Ein verbleibendes, nicht umgesetztes Synthesegas in Form des Stroms m ist durch die Bildung des ersten Recyclestroms p an Kohlendioxid abgereichert. Der Strom m wird in eine nicht dargestellte Aufbereitungseinheit überführt, in der unter Abtrennung eines Stroms o, der insbesondere leichte Inertkomponenten wie Methan oder Wasserstoff enthalten kann, beispielsweise durch Purgen, ein Strom n, der erwähnte zweite Recyclestrom gebildet wird. Der Strom n wird ggf. nachverdichtet damit er das Druckniveau des Synthesegassammelstroms h erhält, ggf. auch in einem Verdichter, der für die Verdichtung des zweiten Synthesegasstroms g verwendet wird, bzw. entsprechenden Verdichterstufen.

Durch den Einsatz des erfindungsgemäßen Verfahrens kann der Synthesegassammelstrom h mit einer Stöchiometriezahl von unter 2 und der Syntheseeinsatzstrom i mit einer Stöchiometriezahl mit über 2 und gleichzeitig geringem Kohlendioxidgehalt erzeugt werden. Es ist aber auch eine Bereitstellung mit einer Stöchiometriezahl von mehr als 2 möglich.

Die Erfindung eignet sich neben den dargestellten Ausführungsformen auch für einen Recycle einer Methanolfraktion aus einer Trenneinheit 6 in die aufbauende Reaktion 5. Weitere Ströme können in einer Trenneinheit 6 oder anderen, nicht veranschaulichten Trenneinheiten anfallen, beispielsweise wasserreiche Ströme. Neben dem ersten Recyclestrom p können auch externe kohlendioxidreiche Ströme der Dampfreformierung 3 zugeführt werden. Derartiges "externes" Kohlendioxid kann beispielsweise aus Erdgas, das als Einsatzstrom a verwendet wird, abgetrennt werden. Entsprechendes Kohlendioxid kann auch in einem Ausgangsstrom a, der zur Bildung der Einsatzströme b und e verwendet wird, verbleiben und daher der partiellen Oxidation bzw. autothermen Reformierung 1 und/oder der Dampfreformierung 3 zugeführt werden.

Der erläuterte Strom o kann, da er wasserstoff- und methanreich ist, beispielsweise als Brenngas verwendet werden, Teile können auch in die Dampfreformierung oder partielle Oxidation 1 zurückgeführt, zu dem Ausgangsstrom a zugespeist oder zu einem nicht dargestellten Prereformer rezykliert werden. Auf diese Weise kann die Energieeffizienz einer entsprechenden Anlage weiter gesteigert werden. Aus dem Strom o kann ferner Wasserstoff, beispielsweise für die Entschwefelung und/oder den Export, gewonnen werden. Der erste Recyclestrom p kann auch teilweise zu einem Synthesegasverdichter, der in Figur 1 ebenfalls nicht veranschaulicht ist, rezykliert werden. Ebenso ist eine Rückführung zu einem weiteren, vorgeschalteten Reaktor, beispielsweise einem Shiftreaktor oder Prereformer, möglich. Auch können entsprechende Ströme zu einem oder mehreren Wärmetauschern rezykliert werden.

**Patentansprüche**

1. Verfahren zur Herstellung eines oder mehrerer Reaktionsprodukte, bei dem ein erster methanreicher Einsatzstrom (b) einem partiellen Oxidationsverfahren und/oder einem autothermen Reformierungsverfahren (1) und ein zweiter methanreicher Einsatzstrom (e) einem Dampfreformierungsverfahren (3) unterworfen wird, und bei dem mittels des partiellen Oxidationsverfahrens und/oder des autothermen Reformierungsverfahrens (1) ein erster Synthesegas enthaltender Abstrom (d) und mittels des Dampfreformierungsverfahrens (3) ein zweiter Synthesegas enthaltender Abstrom (g) gebildet wird, wobei aus Synthesegas des ersten Abstroms (d) und Synthesegas des zweiten Abstroms (g) ein Synthesegassammelstrom (h) gebildet und Fluid des Synthesegassammelstroms (h) in einem Syntheseeinsatzstrom (i) unter Erhalt eines Kohlendioxid und das oder die Reaktionsprodukte enthaltenden Syntheseabstroms (k) einer aufbauenden Reaktion unterworfen wird, **dadurch gekennzeichnet, dass** aus Fluid des Syntheseabstroms (k) zumindest ein kohlendioxidreicher erster Recyclestrom (p) durch Kondensation und Destillation gebildet und bei einem Druck von bis 3 MPa (10 bis 30 bar) bereitgestellt wird, und dass Fluid des ersten Recyclestroms (p) dem Dampfreformierungsverfahren (3) unterworfen wird.

2. Verfahren nach Anspruch 1, bei dem aus Fluid des Syntheseabstroms (k) ein Synthesegas enthaltender und an Kohlendioxid abgereicherter zweiter Recyclestrom (n) gebildet wird, wobei Fluid des zweiten Recyclestroms (n) bei der Bildung des Syntheseeinsatzstroms (i) verwendet wird.

3. Verfahren nach Anspruch 1 oder 2, bei dem der Synthesegassammelstrom (h) eine Stöchiometriezahl von 1,5 bis 2,05 und/oder der Syntheseeinsatzstrom (i) eine Stöchiometriezahl von 2 bis 5 aufweist.

4. Verfahren nach einem der vorstehenden Ansprüche, bei dem aus Fluid des Syntheseabstroms (k) ein an dem oder den Reaktionsprodukten angereicherter Produktstrom (l) gebildet und dem Verfahren entzogen wird.

5. Verfahren nach einem der vorstehenden Ansprüche, bei dem der erste Recyclestrom (p) einen Gehalt von wenigstens 60, 70, 80 oder 90 Molprozent Kohlendioxid aufweist und im Übrigen überwiegend Komponenten mit Siedepunkten unterhalb des oder der Reaktionsprodukte enthält, oder bei dem der erste Recyclestrom (p) ausschließlich aus Kohlendioxid besteht.

6. Verfahren nach einem der Ansprüche 2 bis 4, bei dem der zweite Recyclestrom überwiegend Wasserstoff, Kohlendioxid und Kohlenmonoxid aufweist, wobei der Gehalt an Kohlendioxid von 0 bis 20 Molprozent, insbesondere von 3 bis 15 Molprozent, beträgt.

7. Verfahren nach einem der vorstehenden Ansprüche, bei dem der erste Abstrom (c) auf einem ersten Druckniveau und der zweite Abstrom (f) auf einem zweiten Druckniveau, das geringer als das erste Druckniveau ist, bereitgestellt werden, wobei vor dem Bilden des Synthesegassammelstroms (h) das Synthesegas des zweiten Abstroms (f) auf das erste Druckniveau verdichtet wird.

8. Verfahren nach Anspruch 7, bei dem das erste Druckniveau bei 3 bis 10 MPa (30 bis 100 bar), insbesondere bei 3,5 bis 8 MPa (35 bis 80 bar), und das zweite Druckniveau bei 0,5 bis 4 MPa (5 bis 40 bar), insbesondere bei 1 bis 3,5 MPa (10 bis 35 bar), liegt.

9. Verfahren nach einem der Ansprüche 7 oder 8, bei dem der erste Einsatzstrom (b) und der zweite Einsatzstrom (e) aus Fluid eines Ausgangsstroms (a) gebildet werden, das auf dem ersten und dem zweiten Druckniveau bereitgestellt und zuvor oder anschließend erwärmt und entschwefelt wird.

10. Verfahren nach Anspruch 9, bei dem zum Erwärmen des Fluids des Ausgangsstroms (a) Abwärme des ersten Abstroms (d), des zweiten Abstroms (g) und/oder des Synthesegassammelstroms (h) verwendet wird.

11. Verfahren nach einem der vorstehenden Ansprüche, bei dem die aufbauende Reaktion (5) die Synthese, insbesondere die Direktsynthese, von Dimethylether umfasst und das oder die Reaktionsprodukte Dimethylether umfassen.

**Claims**

1. Process for preparing one or more reaction products, in which a first methane-rich feed stream (b) is subjected to a partial oxidation process and/or an autothermal reforming process (1) and a second methane-rich feed stream (e) is subjected to a steam reforming process (3), and in which a first synthesis gas-containing output stream (d) is formed by means of the partial oxidation process and/or the autothermal reforming process (1) and a second synthesis gas-containing output stream (g) is formed by means of the steam reforming process (3), where synthesis gas from the first output stream (d) and synthesis gas from the second output stream (g) are used to form a collective synthesis gas stream (h) and fluid from the collective synthesis gas stream (h) is subjected to a molecular weight-increasing reaction in a synthesis feed stream (i) to obtain a synthesis output stream (k) comprising carbon dioxide and the reaction product(s), **characterized in that** at least one carbon dioxide-rich first recycle stream (p) is formed by condensation and distillation from fluid from the synthesis output stream (k) and provided at a pressure of 1 to 3 MPa (10 to 30 bar), and **in that** fluid from the first recycle stream (p) is subjected to the steam reforming process (3) .

2. Process according to Claim 1, in which a synthesis gas-containing and carbon dioxide-depleted second recycle stream (n) is formed from fluid from the synthesis output stream (k), using fluid from the second recycle stream (n) in the formation of the synthesis feed stream (i).

3. Process according to Claim 1 or 2, in which the collective synthesis gas stream (h) has a stoichiometric number of 1.5 to 2.05 and/or the synthesis feed stream (i) has a stoichiometric number of 2 to 5.

4. Process according to any of the preceding claims, in which a product stream (1) enriched in the reaction product(s) is formed from fluid from the synthesis output stream (k) and withdrawn from the process.

5. Process according to any of the preceding claims, in which the first recycle stream (p) has a content of at least 60, 70, 80 or 90 mole percent of carbon dioxide and for the rest comprises predominantly components having boiling points below that/those of the reaction product(s), or in which the first recycle stream (p) consists exclusively of carbon dioxide.

6. Process according to any of Claims 2 to 4, in which the second recycle stream comprises predominantly hydrogen, carbon dioxide and carbon monoxide, where the carbon dioxide content is from 0 to 20 mole percent, especially from 3 to 15 mole percent.

7. Process according to any of the preceding claims, in which the first output stream (c) is provided at a first pressure level and the second output stream (f) at a second pressure level lower than the first pressure level, the formation of the collective synthesis gas stream (h) being preceded by compression of the synthesis gas from the second output stream (f) to the first pressure level.

8. Process according to Claim 7, in which the first pressure level is at 3 to 10 MPa (30 to 100 bar), especially at 3.5 to 8 MPa (35 to 80 bar), and the second pressure level at 0.5 to 4 MPa (5 to 40 bar), especially at 1 to 3.5 MPa (10 to 35 bar).

9. Process according to either of Claims 7 and 8, in which the first feed stream (b) and the second feed stream (e) are formed from fluid in a starting stream (a) which is provided at the first and second pressure levels and is previously or subsequently heated and desulfurized.

10. Process according to Claim 9, in which the fluid from the starting stream (a) is heated using waste heat from the first output stream (d), from the second output stream (g) and/or from the collective synthesis gas stream (h).

11. Process according to any of the preceding claims, in which the molecular weight-increasing reaction (5) comprises the synthesis, especially the direct synthesis, of dimethyl ether and the reaction product(s) comprise(s) dimethyl ether.

**Revendications**

1. Procédé de fabrication d'un ou de plusieurs produits de réaction, selon lequel un premier courant de charge riche en méthane (b) est soumis à un procédé d'oxydation partielle et/ou à un procédé de reformage autotherme (1), et un deuxième courant de charge riche en méthane (e) est soumis à un procédé de reformage à la vapeur (3), et selon lequel un premier courant de sortie (d) contenant un gaz de synthèse est formé au moyen du procédé d'oxydation partielle et/ou du procédé de reformage autotherme (1), et un deuxième courant de sortie (g) contenant un gaz de synthèse est formé au moyen du procédé de reformage à la vapeur (3), un courant collectif de gaz de synthèse (h) étant formé à partir du gaz de synthèse du premier courant de sortie (d) et du gaz de synthèse du deuxième courant de sortie (g), et le fluide du courant collectif de gaz de synthèse (h) étant soumis dans un courant de charge de synthèse (i) à une réaction d'augmentation du poids moléculaire pour obtenir un courant de sortie de synthèse (k) contenant du dioxyde de carbone et le ou les produits de réaction, **caractérisé en ce qu'**au moins un premier courant de recyclage riche en dioxyde de carbone (p) est formé par condensation et distillation à partir du fluide du courant de sortie de synthèse (k), et mis à disposition à une pression de 1 à 3 MPa (10 à 30 bar), et **en ce que** le fluide du premier courant de recyclage (p) est soumis au procédé de reformage à la vapeur (3).

2. Procédé selon la revendication 1, selon lequel un deuxième courant de recyclage (n) contenant un gaz de synthèse et appauvri en dioxyde de carbone est formé à partir du fluide du courant de sortie de synthèse (k), le fluide du deuxième courant de recyclage (n) étant utilisé lors de la formation du courant de charge de synthèse (i).

**3.** Procédé selon la revendication 1 ou 2, selon lequel le courant collectif de gaz de synthèse (h) présente un coefficient stoechiométrique de 1,5 à 2,05 et/ou le courant de charge de synthèse (i) présente un coefficient stoechiométrique de 2 à 5.

**4.** Procédé selon l'une quelconque des revendications précédentes, selon lequel un courant de produits (1) enrichi en le ou les produits de réaction est formé à partir du fluide du courant de sortie de synthèse (k) et extrait du procédé.

**5.** Procédé selon l'une quelconque des revendications précédentes, selon lequel le premier courant de recyclage (p) présente une teneur d'au moins 60, 70, 80 ou 90 pour cent en moles en dioxyde de carbone, et contient pour le reste principalement des composants ayant des points d'ébullition inférieurs au ou aux produits de réaction, ou selon lequel le premier courant de recyclage (p) est exclusivement constitué par du dioxyde de carbone.

**6.** Procédé selon l'une quelconque des revendications 2 à 4, selon lequel le deuxième courant de recyclage comprend principalement de l'hydrogène, du dioxyde de carbone et du monoxyde de carbone, la teneur en dioxyde de carbone étant de 0 à 20 pour cent en moles, notamment de 3 à 15 pour cent en moles.

**7.** Procédé selon l'une quelconque des revendications précédentes, selon lequel le premier courant de sortie (c) est mis à disposition à un premier niveau de pression et le deuxième courant de sortie (f) est mis à disposition à un deuxième niveau de pression, qui est inférieur au premier niveau de pression, le gaz de synthèse du deuxième courant de sortie (f) étant comprimé au premier niveau de pression avant la formation du courant collectif de gaz de synthèse (h).

**8.** Procédé selon la revendication 7, selon lequel le premier niveau de pression est de 3 à 10 MPa (30 à 100 bar), notamment de 3,5 à 8 MPa (35 à 80 bar), et le deuxième niveau de pression est de 0,5 à 4 MPa (5 à 40 bar), notamment de 1 à 3,5 MPa (10 à 35 bar).

**9.** Procédé selon l'une quelconque des revendications 7 ou 8, selon lequel le premier courant de charge (b) et le deuxième courant de charge (e) sont formés à partir du fluide d'un courant de départ (a), qui est mis à disposition au premier et au deuxième niveau de pression et, précédemment ou ultérieurement, chauffé et désulfuré.

**10.** Procédé selon la revendication 9, selon lequel la chaleur résiduaire du premier courant de sortie (d), du deuxième courant de sortie (g) et/ou du courant collectif de gaz de synthèse (h) est utilisée pour le chauffage du fluide du courant de départ (a).

**11.** Procédé selon l'une quelconque des revendications précédentes, selon lequel la réaction d'augmentation du poids moléculaire (5) comprend la synthèse, notamment la synthèse directe, d'éther diméthylique, et le ou les produits de réaction comprennent de l'éther diméthylique.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2362944 A1
- DE 102012001811
- US 20090105356 A1
- US 7485767 B2
- WO 1993015999 A1
- EP 0522744 A2

**In der Beschreibung aufgeführte Nicht-Patentliteratur**

- Gas Production. Ullmann's Encyclopedia of Industrial Chemistry. Onlineausgabe, 15. Dezember 2006